# FASCICULE DE BREVET EUROPEEN

(11) **EP 0 850 641 B1**
(45) Date de publication et mention de la délivrance du brevet: **18.06.2003**
(21) Numéro de dépôt: 97402966.2
(22) Date de dépôt: 08.12.1997
(51) Int. Cl.: A61K 7/48

(54) **Utilisation des sophorolipides en tant qu'agent stimulateur du métabolisme des fibroblastes dermiques**
Anwendung von Sophorolipiden als Stimulierungsmittel des Metabolismus von Hautfibroblasten
Use of Sophorolipides as a stimulating agent of the metabolism of dermic fibroblasts

(30) Priorité: 27.12.1996 FR 9616093
(43) Date de publication de la demande: 01.07.1998
(73) Titulaire: SOPHOR S.A., 92500 Reuil-Malmaison (FR)
(72) Inventeur: Borzeix, Frédérique, 92500 Rueil-Malmaison (FR)

(56) Documents cités:
- EP-A- 0 209 783
- WO-A-95/34282
- DATABASE WPI Section Ch, Week 8008 Derwent Publications Ltd., London, GB; Class D16, AN 80-13763C XP002041981 & JP 55 004 344 A (KAO SOAP CO LTD) , 12 janvier 1980

## Description

L'invention concerne une utilisation d'au moins un sophorolipide en tant qu'agent stimulateur du métabolisme des cellules de fibroblastes du derme de la peau. Elle s'applique particulièrement au domaine de la cosmétologie.

Les propriétés des sophorolipides concernant la lutte contre le vieillissement cutané à savoir leur action antiradicalaire et anti-élastasique ont été décrites dans la demande de brevet W095/34282. Par ailleurs, l'arrière plan technologique est illustré par les brevets EP-A209 783 et la publication Database WPI, Section Ch, WEEK 8008, Class D16, AN 80-13763C (JP 55 004 344A). En aucun cas les propriétés concernant la stimulation du métabolisme des fibroblastes que l'on se propose de développer ici n'ont été décrites ni suggérées.

Les propriétés mécaniques de la peau sont en grande partie assurées par les fibres de collagène qui constituent la trame principale de la matrice du derme.

Les collagènes constituent une famille d'une vingtaine de protéines distinctes dont la moitié est représentée dans le derme. Ce sont des protéines riches en proline et hydroxyproline, synthéthisées par les fibroblastes.

Lors du vieillissement, le métabolisme et/ou la structure des collagènes peuvent être modifiés. Une diminution de la synthèse des collagènes et une augmentation de la réticulation des fibres sont observées. Par ailleurs, lors du vieillissement cutané, le renouvellement cellulaire est ralenti.

Des produits d'origine animale tels que les extraits embryonnaires, les extraits de glandes mammaires, les extraits placentaires, le liquide amniotique, ont été largement utilisés dans des produits de soins pour leurs propriétés de stimulation de l'activité cellulaire et donc leur action préventive contre le vieillissement cutané.
Avec l'apparition en 1986 de la maladie de la vache folle, ces matières premières ne sont plus utilisées en industrie cosmétique de nos jours.

Aussi, des produits tels que les oligoéléments comme le zinc, le cuivre, le manganèse, peuvent stimuler la prolifération fibroblastique et augmenter notamment la production de collagène par les fibroblastes.

D'autre part, l'acide ascorbique (vitamine C) permet d'augmenter la synthèse des collagènes. Il faut noter que la fragilité des vitamines rend leur incorporation en l'état dans des produits finis, délicate. Par ailleurs, leur stabilité au stockage, à la chaleur, au pH ou encore à la lumière est limitée.

Par conséquent, l'utilisation de produits naturels d'origine végétale ne présentant pas de problème de stabilité et de surcroît, faciles d'incorporation en formulation, est recherchée à ce jour dans les domaines de la cosmétologie et de la dermocosmétique. C'est l'un des objets de l'invention.

A ce titre, les sophorolipides, glycolipides d'origine naturelle, obtenus par fermentation levurienne, répondent aux besoins attendus. En effet, des études *in vitro* ont montré que les sophorolipides ont une action bénéfique sur la synthèse des collagènes par les fibroblastes dermiques et sont de bons agents stimulants de la division cellulaire.

Leur efficacité comparée à celles du sérum de veau foetal et de la vitamine C a été démontrée *in vitro*. Leur action en tant qu'agent restructurant et réparateur du tissu conjonctif en fait des produits actifs contre le vieillissement cutané. Plus précisément, les sophorolipides stimulent la synthèse de collagène *in vitro,* ce qui permet pour des applications cosmétologiques de les utiliser en tant qu'agent restructurant de la peau.

Les sophorolipides stimulent significativement les cellules de la peau humaine et de ce fait favorisent la reconstruction du tissu conjonctif. On peut donc les utiliser en tant qu'agent réparateur de la peau. Enfin, l'augmentation de l'adhérence des cellules de fibroblastes sur des supports de culture en présence de sophorolipides, permet de justifier leur utilisation en tant qu'agent raffermissant de la peau (fermeté cutanée). Bien évidemment, ils peuvent présenter l'ensemble de ces propriétés.

L'ensemble de ces propriétés font que les sophorolipides, en favorisant la reconstruction du tissu conjonctif, peuvent avoir une action réparatrice au même titre qu'une action préventive contre le vieillissement cutané.

Les sophorolipides forme acide pourront être utilisés dans des gammes de concentration variant de 1 ppm à 10 % en poids sur volume (p/v) en matière active en formulation et notamment dans des émulsions de type eau/huile et huile/eau (laits, crèmes) ou dans des gels et des sérums. Leur utilisation se fera plus particulièrement dans des gammes de concentration variant de 0,02 % à 1 % en matière active. Les sophorolipides forme brute (mélange de forme acide et de forme lactone) pourront être utilisés à des concentrations variant de 0,05 ppm à 6 % en matière active et plus particulièrement de 80 ppm à 2 %.

Par forme acide, on entend la forme acide protonée et/ou forme acide au moins en partie sous forme de sels métalliques monovalents ou divalents.
Les sophorolipides peuvent être utilisés sous une forme dérivée sensiblement dans les mêmes concentrations que celles des sophorolipides forme acide, par exemple sous la forme ester. Les sophorolipides ont leur fonctions acide au moins en partie estérifiée par réaction avec un alcool linéaire ou ramifié de 1 à 18 atomes de carbone et de préférence 1 à 8 atomes de carbone.

Les sophorolipides forme brute sont obtenus par fermentation selon le procédé décrit dans le brevet FR 2670 798 incorporé comme référence, concernant la production de sophorolipides par fermentation avec alimentation continue (Fed batch) en ester d'acides gras ou en huile.
Les sophorolipides sous forme acide désacétylée sont obtenus après hydrolyse alcaline des sophorolipides forme brute et neutralisation (Davila et al., 1993 - Journal of chromatography 648,139-149).
L'acide acétique résiduel contenu dans le produit d'hydrolyse est éliminé selon une méthode classique de séparation c'est à dire par passage sur résine anionique de type IRA 93.

Les sophorolipides sont des glycolipides d'origine végétale, parfaitement biodégradables. Leur structure est constituée d'une fraction glucidique représentée par un dimère de glucose, le sophorose (2-O-β-D-glucopyranosyl, β-D-glucopyranose) relié par une liaison acétale à un hydroxyacide gras en position terminale ou subterminale.
Les hydroxyacides gras les plus représentés comportent 16 à 18 atomes de carbone et peuvent être saturés mono ou di-insaturés. L'hydroxyacide gras majoritairement incorporé dans les sophorolipides et ce, indépendamment du substrat lipidique utilisé, est l'acide 17-hydroxyoléique (tableau 1).

Les sophorolipides sont constitués d'un mélange de glycolipides de structures voisines qui diffèrent les unes des autres par une lactonisation ou non du sophorose (principalement en position 4") ou encore par le degré d'acétylation de ce dernier.
La forme majoritairement représentée (≥ 80 %) dans les sophorolipides forme brute est la forme lactonique.

**Tableau 1**

| Répartition des hydroxyacides gras constitutifs des sophorolipides obtenus par fermentation à partir de glucose et d'ester d'huile de colza | |
|---|---|
| Acides gras | % |
| 15-OH C16 : 0 | 1,7 |
| 16-OH C16 : 0 | 1,2 |
| 17-OH C18 : 2 | 6,7 |
| 17-OH C18 : 1 | 63,4 |
| 17-OH C18: 0 | 3,2 |
| 18-OH C18 : 2 | 13,5 |
| 18-OHC18: 1 | 1,03 |

Les exemples suivants illustrent l'invention.

### EXEMPLE 1 Effet des sophorolipides forme acide sur la néosynthèse des collagènes. Etude in vitro en système monocouche

On s'intéresse ici à l'effet des sophorolipides forme acide sur une des étapes du métabolisme des collagènes, celle de la néosynthèse.

Un modèle *in vitro,* reposant sur l'utilisation de fibroblastes de derme humain cultivés dans des lattices de collagène (derme équivalent) a été retenu pour évaluer les effets des sophorolipides forme acide.
Les molécules de collagènes contiennent une forte proportion de proline, c'est donc l'incorporation de la proline dans les protéines, essentiellement dans les collagènes, qui a été mesurée.

Les spécifications des sophorolipides forme acide sont données ci-après :
. matière sèche : ≤ 25 % ± 2 %
. pH : 6 ± 0,5
. minéraux : ≤ 1 %
. sucres libres : ≤ 1 %
. acides gras libres : ≤ 0,5 %
. acétate : ≤ 1 %

Les produits de référence pour la réalisation des essais sont :
. ascorbate de sodium (vitamine C)
. sérum de veau foetal (SVF)

Les cellules de fibroblastes obtenus à partir d'explants du derme ont été cultivées et employées au septième passage.
Les systèmes d'essai sont constitués par des lattices de collagène contractées par les fibroblastes après 96 heures de culture.

Les sophorolipides forme acide ou salifiée ainsi que les molécules de référence sont directement dissous dans du milieu d'incubation puis mis en présence des systèmes d'essai dans lesquels on a ajouté du milieu d'incubation contenant de la proline tritiée.
Les lattices sont ensuite incubées 72 heures à 37°C, sous atmosphère contenant 5 % de CO₂.

La mesure de l'incorporation de la proline tritiée est réalisée de la façon suivante :
Les lattices ont été incubées pendant une heure, sous agitation douce et à température ambiante. Les échantillons ont ensuite été placés dans des fioles à scintillation.

La radioactivité a été mesurée avec un compteur PACKARD. Les valeurs ont été exprimées en cpm par lattice.

Des cultures témoins (sans produit à l'essai) ont été réalisées en parallèle. Chaque condition expérimentale a été effectuée en triplicate. Les groupes de données (groupe témoin et groupes traités) ont été traités par une analyse de la variance à un facteur (ANOVA 1), suivie par un test de Dunnett (p = probabilité de se trouver en dehors de la marge d'erreur).

Les effets des sophorolipides forme acide sur la synthèse des collagènes ont été étudiés dans un modèle de derme équivalent. Celui-ci était constitué par des lattices de collagène contractées par des fibroblastes dermiques d'une femme âgée de 23 ans.

La vitamine C et le sérum de veau foetal ont été utilisés comme produits de référence. Le produit à l'essai et les produits de référence ont été dilués dans le milieu d'incubation et incubés avec les dermes équivalents pendant 72 heures.

Les effets sur la synthèse des collagènes ont été évalués par la mesure de l'incorporation de ³H-proline dans les protéines néosynthétisées par les fibroblastes. Dans le modèle utilisé, l'incorporation de la proline tritiée dans les protéines a lieu principalement dans les collagènes.

L'évaluation des effets des sophorolipides forme acide et des produits de référence sur la néosynthèse des collagènes montre que :
- le sérum de veau foetal à 10 % (v/v) augmente d'un facteur 4,1 la synthèse des collagènes (tableau 2).
- la vitamine C à 1 mg/ml augmente d'un facteur 1,8 l'incorporation de proline tritiée dans les protéines néosynthétisées (tableau 2).
- les sophorolipides forme acide testés à 0,2 ; 1 et 5 mg/ml augmentent significativement l'incorporation de proline tritiée dans les protéines néosynthétisées d'un facteur 2 ; 1,5 et 1,6 respectivement (p<0,05) (tableau 3).

Les sophorolipides forme acide ou salifiée stimulent la synthèse de collagène dans ce modèle de derme équivalent et montrent un effet restructurant de la peau.

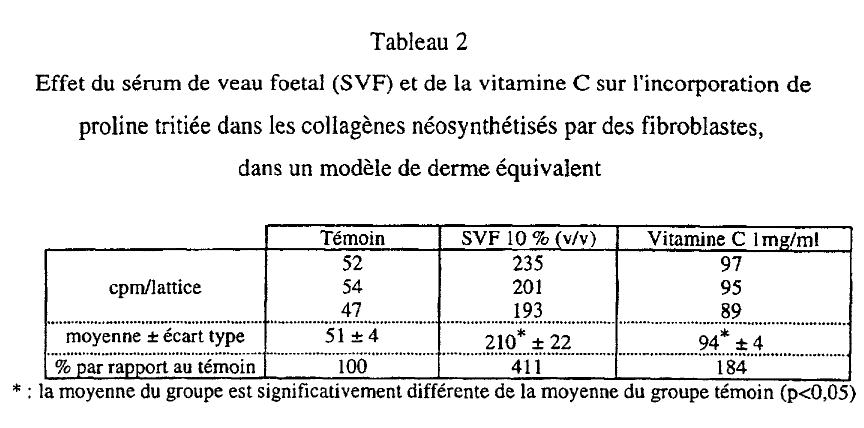

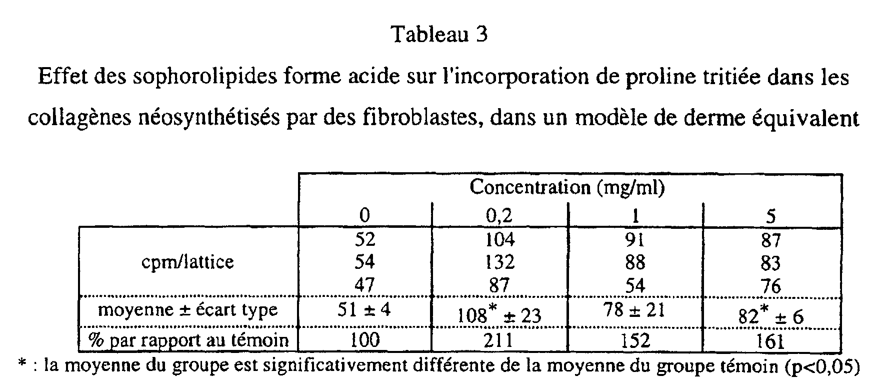

### EXEMPLE 2 : Effet des sophorolipides forme brute sur la synthèse des collagènes

### Modèle monocouche : fibroblastes dermiques humains cultivés en lattices

La réalisation expérimentale est semblable à celle décrite dans l'exemple 1.

L'étude a été pratiquée ici sur les sophorolipides forme brute.

Les spécifications des sophorolipides forme brute sont données ci-après :
. matière sèche : ≤ 30 % ± 2 %
. pH : 7 ± 0,5
. minéraux : ≤ 2 %
. sucres libres : ≤ 1 %
. acides gras libres : ≤ 3 %
. cendres : ≤ 0,04 %
. densité : 1,03 à 20°C
. viscosité : 115,3 mPa. s à 20°C
   62,4 mPa. s à 40°C

L'évaluation des effets des sophorolipides forme brute sur synthèse des collagènes dans un modèle de derme équivalent montre que :
- les sophorolipides forme brute testés à 0,08 µg/ml ; 0,4 µg/ml et 2 µg/ml augmentent significativement l'incorporation de proline tritiée dans les protéines néosynthétisées d'un facteur 2,3 ; 2,2 et 2 respectivement (p<0,05) (tableau 4)
   Ces résultats ne montrent pas d'effet dose du produit à l'essai, la stimulation maximale étant obtenue dès la concentration égale à 0,08 µg/ml.

Les sophorolipides forme brute stimulent donc la synthèse de collagène dans ce modèle de derme équivalent et montrent un effet restructurant de la peau.

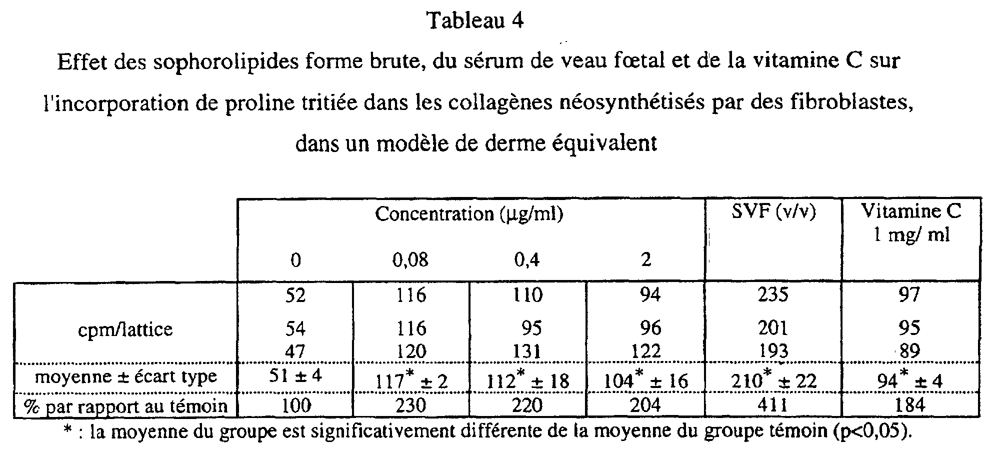

### EXEMPLE 3 Effet des sophorolipides forme acide sur la synthèse des collagènes et la division cellulaire. Etude in vitro dans un modèle de peau reconstruite

On s'intéresse ici à un modèle expérimental SKIN² correspondant à une peau reconstruite avec un épiderme et un derme.

De façon à évaluer l'effet des sophorolipides forme acide sur la synthèse des collagènes, l'incorporation de proline tritiée dans les protéines acido-solubles a été mesurée. D'autre part, la viabilité des peaux SKIN² à la fin du traitement a été mesurée.

Les sophorolipides forme acide, dont les spécifications ont été décrites dans l'exemple 1, ont été testés à 1 et 10 mg/ml.
Le mélange composé de 0,2 mg/ml de vitamine C et de 10 % (v/v) de SVF a été utilisé comme référence positive.
Les peaux reconstituées SKIN² ont été utilisées conformément aux instructions du fabricant.
Les produits directement dissous dans le milieu d'essai ont été appliqués sur l'épiderme une heure par jour pendant trois jours consécutifs.
Les effets sur la synthèse de collagène ont été évalués par mesure de l'incorporation de ³H-proline dans les protéines néosynthétisées par les fibroblastes. Cette incorporation a lieu principalement dans les molécules de collagène.

La viabilité des peaux SKIN² a été contrôlée par la technique colorimétrique au MTT (bromure de diméthyl-thiazol diphényl tétrazolium).
Les résultats montrent que le mélange vitamine C + SVF n'est pas cytotoxique (tableau 5) et qu'il augmente d'un facteur 1,3 l'incorporation de proline tritiée dans les protéines néosynthétisées (tableau 5).
Les sophorolipides forme acide testés à 1 et 10 mg/ml ne sont pas cytotoxiques vis-à-vis des peaux SKIN² (tableau 5).
A 1 et 10 mg/ml, ils augmentent d'un facteur 1,4 et 1,2 respectivement la réduction du MTT par les cellules. Ceci peut correspondre à une augmentation du métabolisme cellulaire à nombre égal de cellules, ou à une augmentation du nombre de cellules, c'est à dire une stimulation de la division cellulaire.
A 10 mg/ml, ils augmentent de 14 % l'incorporation de proline tritiée dans les protéines néosynthétisées (tableau 6).
La moindre amplitude d'augmentation observée dans ce travail par rapport aux résultats obtenus dans le modèle monocouche peut s'expliquer par une capacité de réponse moindre dans le modèle SKIN² par rapport aux fibroblastes en monocouche : le facteur de stimulation obtenu avec le SVF était de 1,3 dans le modèle SKIN² contre 4 dans le modèle monocouche (exemple 1).

Le métabolisme cellulaire est différent dans les deux modes de culture : dans le modèle SKIN², les fibroblastes restent capables de division, ce qui n'est pas le cas en monocouche et il est bien connu que les cellules en phase de division sont moins différenciées et synthétisent moins de protéines de la matrice extracellulaire.

Ces éléments pourraient expliquer que le potentiel de stimulation des sophorolipides se traduise différemment dans les deux situations : par une augmentation du métabolisme cellulaire et/ou du nombre de cellules dans SKIN² et par une augmentation de la néosynthèse des protéines en monocouche. Dans la peau à l'état normal, les fibroblastes sont confluents et c'est plutôt le paramètre "collagène" qui serait influencé alors que dans une peau qui se remodèle (dans un état de cicatrisation par exemple), c'est plutôt le paramètre "division" qui serait augmenté.
En tout état de cause, les sophorolipides forme acide, dans les deux études réalisées, s'avèrent être un stimulant significatif des cellules de la peau humaine, dans des gammes de concentrations faibles.

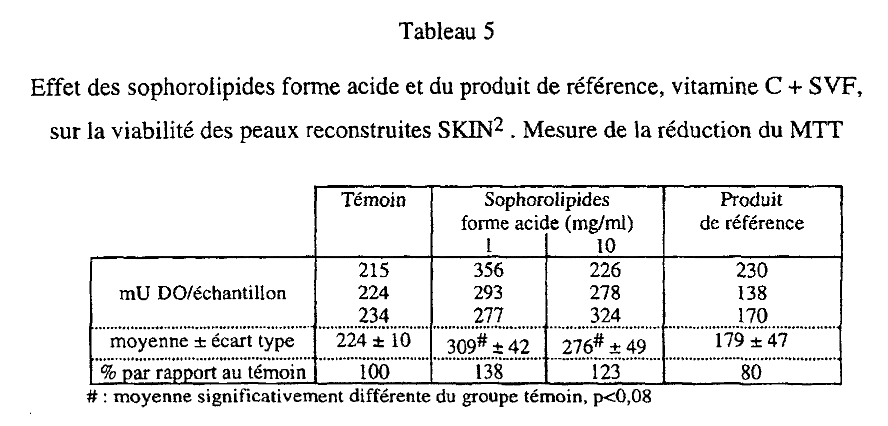

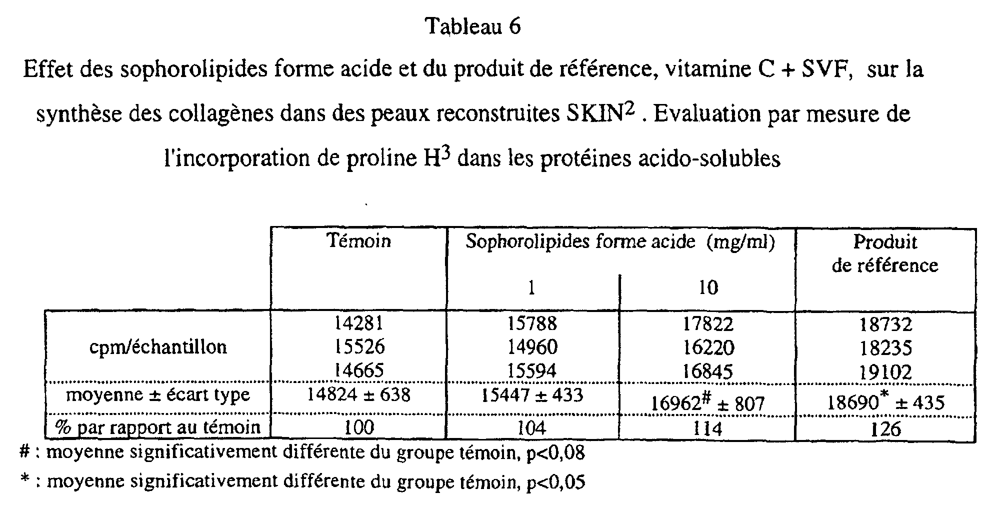

### EXEMPLE 4: Effet des sophorolipides forme brute sur la synthèse des collagènes et la division cellulaire - Modèle de peau reconstruite SKIN²

On s'intéresse ici aux effets des sophorolipides forme brute sur la synthèse des collagènes et la division cellulaire dans un modèle de peau reconstruite.
Les conditions expérimentales sont identiques à celles décrites dans l'exemple 3. Les spécifications des sophorolipides forme brute utilisés sont celles décrites dans l'exemple 2.
Les résultats ont montré que les sophorolipides forme brute à 0,1 ; 1 ; 10 et 100 µg/ml, n'étaient pas cytotoxiques vis-à-vis des peaux SKIN² (tableau 7). A 0,1 µg/ml, ils augmentent d'un facteur 1,6 la réduction du MTT par les cellules. Ceci peut correspondre à une augmentation du métabolisme cellulaire à nombre de cellules égal, ou à une augmentation du nombre de cellules, c'est à dire une stimulation de la division cellulaire. Les sophorolipides forme brute à 1 et à 10 µg/ml, augmentent de 10 % l'incorporation de proline tritiée (tableau 8).

De la même façon que dans le cas des sophorolipides forme acide, il semble que le potentiel de stimulation des sophorolipides forme brute se traduise différemment dans les deux situations : par une augmentation du métabolisme cellulaire et/ou du nombre de cellules dans SKIN² et par une augmentation de la néosynthèse des protéines en monocouche ( exemple 2). Dans la peau à l'état normal, les fibroblastes sont confluents et c'est plutôt le paramètre "collagène" qui serait influencé alors que dans une peau qui se remodèle (dans un état de cicatrisation par exemple) c'est plutôt le paramètre "division" qui serait augmenté.
En tout état de cause, les sophorolipides forme brute, dans les deux études réalisées, s'avèrent être des stimulants significatifs des cellules de la peau humaine, dans des gammes de concentrations faibles.

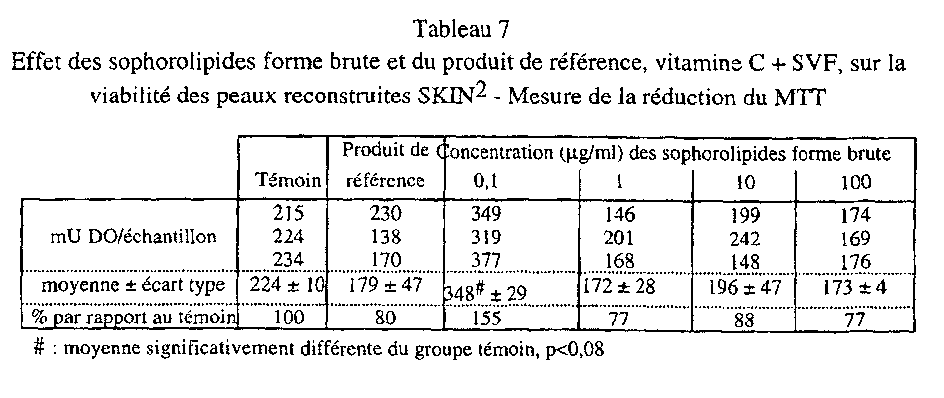

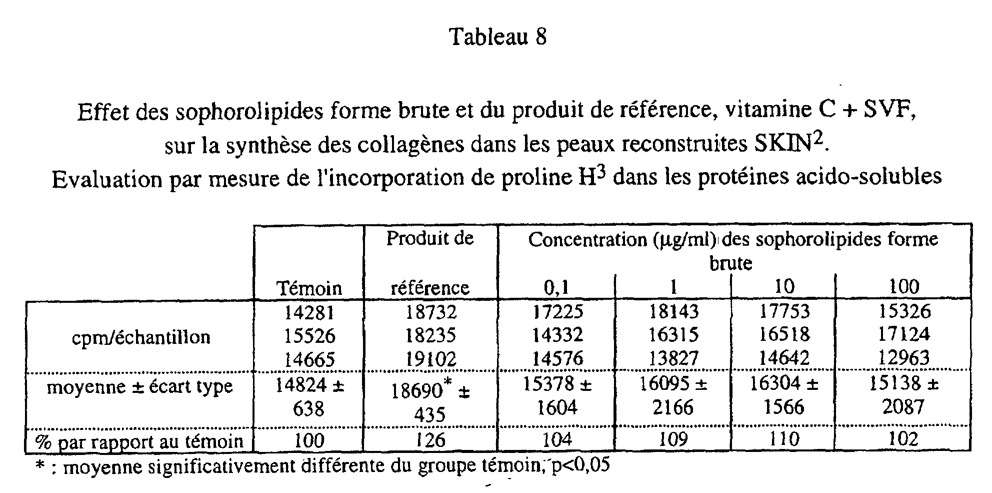

### EXEMPLE 5 : Effet des sophorolipides forme acide sur les propriétés d'adhérence des fibroblastes dermiques humains

On se propose d'évaluer les propriétés des sophorolipides forme acide vis-à-vis des relations entre les fibroblastes du derme cutané et leur environnement. Plus précisément, les effets du produit sur l'adhérence des fibroblastes à une surface de culture recouverte ou non par du collagène ont été mesurés.

Les fibroblastes obtenus par culture d'explants de peau ont été employés au douzième passage. Des plaques de microtitration de 24 puits non traitées pour la culture ont été utilisées pour les essais, recouvertes ou non par du collagène.

Les sophorolipides forme acide testés à 0,2 ; 1 et 5 mg/ml ainsi que le sérum de veau foetal testé à 2 % et 10 % (v/v) sont dilués dans du milieu contenant des fibroblastes à raison de 75000 cellules par puits et incubés à 37°C. Le nombre de cellules adhérentes dans les puits de culture a été évalué par la méthode de réduction du MTT (mesure de la viabilité cellulaire).

Les effets des sophorolipides forme acide sur l'adhérence des fibroblastes dermiques humains à une surface plastique recouverte ou non par du collagène ont été évalués. Le sérum de veau foetal a été utilisé comme produit de référence.

Les résultats montrent que :
a) sur un support plastique hydrophobe, non traité pour la culture de cellules et non recouvert de collagène
   - en présence de SVF à 2 % (v/v), le nombre de cellules adhérentes était comparable au témoin après 25 minutes d'incubation. Le SVF à 10 % (v/v) augmentait d'un facteur 1,2 le nombre de cellules adhérentes (tableau 9). Cet effet validait l'essai.
   - les sophorolipides testés à 0,2 mg/ml augmentaient d'un facteur 1,5 le nombre de cellules adhérentes. A 1 et 5 mg/ml, l'adhérence des cellules sur le support était comparable à celle du témoin (tableau 9).
b) sur un support de culture, non traité pour la culture de cellules et recouvert de collagène
   - en présence de SVF à 2 et 10 % (v/v), le nombre de cellules adhérentes était comparable au témoin après 25 minutes d'incubation (tableau 10). Cet effet validait l'essai.
   - les sophorolipides testés à 0,2 mg/ml augmentaient d'un facteur 1,4 le nombre de cellules adhérentes. A 1 mg/ml et 5 mg/ml, l'adhérence des cellules sur le support était comparable à celle du témoin (tableau 10).

En conclusion, dans cette étude *in vitro,* les sophorolipides forme acide augmentent l'adhérence des fibroblastes sur le support de culture. Ces résultats pourraient indiquer une modification par les sophorolipides de la membrane cellulaire ou des composants de celle-ci et une modulation des relations des fibroblastes dermiques avec l'environnement matriciel en particulier. Ces données permettent de revendiquer une contribution des sophorolipides forme acide à la fermeté cutanée. De manière générale, il apparaît que la forme acide ou salifiée s'avère particulièrement intéressante du fait d'une cytotoxicité plus faible que celle de la forme brute.

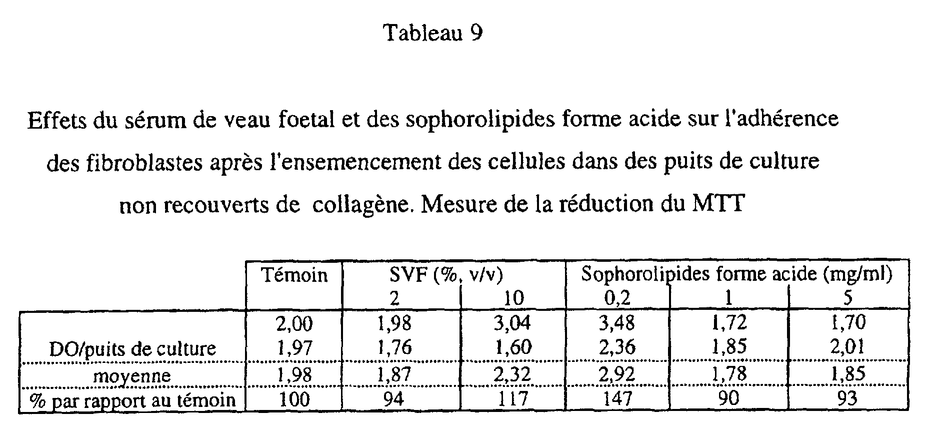

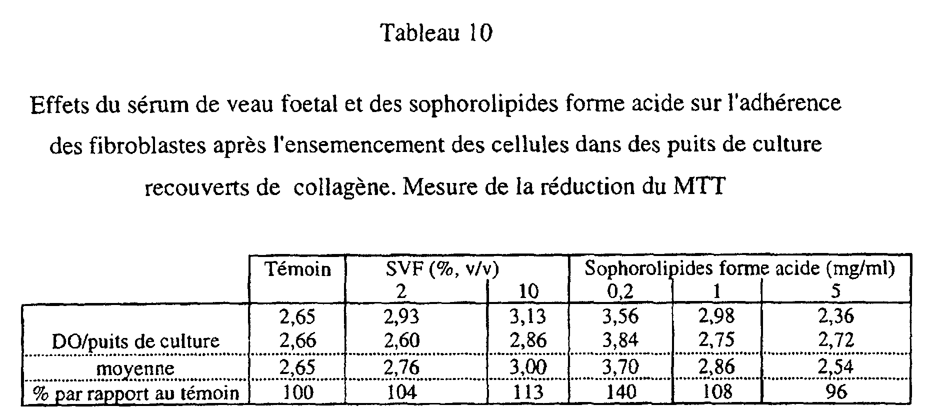

### EXEMPLE 6: Effet des sophorolipides forme brute sur les propriétés d'adhérence des fibroblastes dermiques humains

Les conditions expérimentales sont identiques à celles décrites dans l'exemple 5. Les spécifications des sophorolipides forme brute sont celles décrites dans l'exemple 2.

Les effets des sophorolipides forme brute sur l'adhérence des fibroblastes dermiques humains sur une surface plastique recouverte ou non par du collagène ont été évalués. Le sérum de veau foetal a été utilisé comme produit de référence.

Les résultats montrent que :
a) sur un support plastique hydrophobe, non traité pour la culture de cellules et non recouvert de collagène
   - Les sophorolipides forme brute testés à 0,08 et 0,4 µg/ml augmentaient d'un facteur 1,3 et 1,2 respectivement le nombre de cellules adhérentes au support plastique hydrophobe. A 2 µg/ml, l'adhérence des cellules sur ce support était comparable à celle du témoin (tableau 11).
b) sur un support de culture non traité, pour la culture de cellules et recouvert de collagène
   - Les sophorolipides forme brute testés à 0,08 µg/ml et 0,4 µg/ml augmentaient d'un facteur 1,3 le nombre de cellules adhérentes au support plastique hydrophobe. A 2 µg/ml, l'adhérence des cellules sur ce support était comparable à celle du témoin (tableau 12).

Dans cette étude *in vitro,* les sophorolipides forme brute augmentaient l'adhérence des fibroblastes sur le support de culture. Ces résultats pourraient indiquer une modification par les sophorolipides forme brute de la membrane cellulaire ou des composants de celle-ci et une modulation des relations des fibroblastes dermiques avec l'environnement matriciel en particulier. Ces données permettent de revendiquer une contribution des sophorolipides forme brute à la fermeté cutanée.

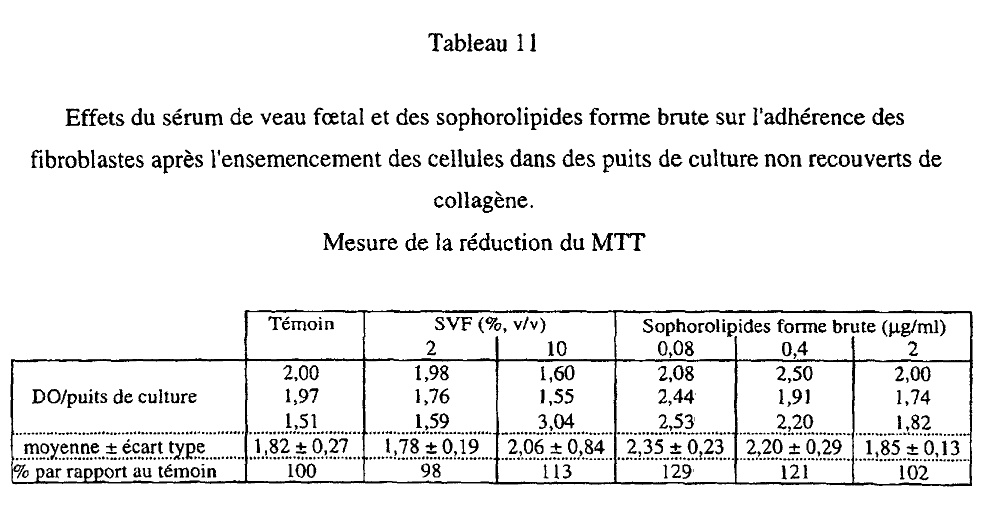

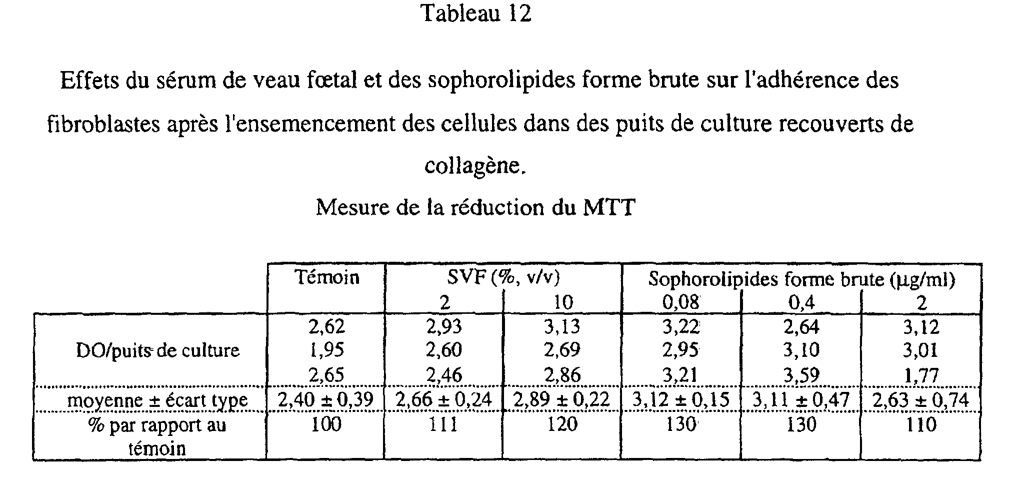

### EXEMPLE 7 : Formulations cosmétiques à base de sophorolipides

Les sophorolipides forme acide ou brute, peuvent être à la fois introduits dans la phase huileuse et dans la phase aqueuse.
Si l'on souhaite les introduire dans la phase huileuse, il faut les dissoudre dans la phase huileuse à 70°C, juste avant l'émulsification dans la phase aqueuse.
Dans les crèmes, gel, sérum, les sophorolipides peuvent être introduits en fin de fabrication, à froid.

### 1 Crème hydratante aux sophorolipides émulsion du type huile dans eau

| | | |
|---|---|---|
| A | - Sophorolipides forme brute ou acide | 0,6 % |
| | - Phenonip | 0,5 % |
| | - Parfum | 0,2 % |
| B | - Stéarate de glycérol | 4 % |
| | - PEG 150 distearate | 0,5 % |
| | - Alcool cétéaryl | 2 % |
| | - Huile de germe de tournesol | 2 % |
| | - Octanoate cétéaryl | 1 % |
| | - Triglycérides | 8 % |
| | - Diméthicone | 0,5 % |
| C | - Eau | 57,10 % |
| D | - Glycérine | 3 % |
| | - Carbomer 840 | 0,3 % |
| | - Triéthanolamine | 0,3 % |
| | - Eau | 20 % |

- Préparer la phase D : ajouter dans l'ordre la glycérine, le carbomer et l'eau puis la triethanolamine.
- Faire chauffer séparément B et C (65°C) et ajouter progressivement B dans C sous agitation.
- Ajouter D, diminuer la température à 30-35°C, ajouter alors les sophorolipides ainsi que le conservateur et le parfum.

### 2 Emulsions de type eau/huile

Les formules sont les suivantes :

### Crème eau/huile

- Huile minérale 19,00 %
- Polyglycéryl-3 diisostéarate 5,00 %
- Glycérine 5,00 %
- Acrylate Copolymère 3,00 %
- Sophorolipides forme acide 1 %
- Sodium chlorure 0,80 %
- Magnésium sulfate 0,80 %
- Phénonip 0,50 %
- Parfum 0,20 %
- Eau QSP 100 %

### 3 Gel démaquillant

| | | |
|---|---|---|
| A | - Sophorolipides | 1 % |
| B | - Hydroxypropyl Guar | |
| | Hydroxypropyl trimonium chloride | 0,3 % |
| | - Hydroxyéthyl cellulose | 1 % |
| | - Eau | 86,5 % |
| C | - Sodium méthyl cocoyl taurat | 0,4 % |
| | - Eau QSP | 100 % |
| D | - Phénonip | 0,5 % |
| | - Nonoxynol 10 | 0,2 % |
| | - Parfum | 0,10 % |

- Mélanger la phase C en chauffant modérément.
- Hydrater la phase B et y ajouter progressivement la phase C.
- Préparer la phase D, la mélanger à la préparation BC et enfin ajouter les sophorolipides.
- Mélanger jusqu'à l'obtention d'un gel homogène.

### 4 Sérum aux sophorolipides

| | | |
|---|---|---|
| A | - Sophorolipides forme brute ou forme acide | 1 % |
| | - Phénonip | 0,5 % |
| B | - EDTA | 0,04% |
| | - Sodium sulfite | 0,01 % |
| | - Sodium disulfite | 0,01 % |
| | - Eau QSP | 100 % |
| C | - Gomme xanthane | 0,04 % |
| | - Hydroxyéthylcellulose | 0,24 % |

- Préparer la phase B. Hydrater C avec B puis ajouter A. Mélanger en évitant la formation de bulles d'air.

### 5 Gel contour des yeux aux sophorolipides

| | | |
|---|---|---|
| A | - Sepigel 305 (Seppic) | 5 % |
| | - Silicone V30 | 1 % |
| B | - Sophorolipides forme brute | 0,6 % |
| | - Polysorbate 20 | 5 % |
| | - Cetiol HE (Henkel) | 3 % |
| | - Phénonip | 0,6 % |
| | - Parfum | 0,04 % |
| C | - Eau QSP | 100 % |

- Préparer séparément les phases A et B. Ajouter C dans B tout en agitant. Lorsque le mélange est homogène, l'ajouter à la phase A en agitant vigoureusement. Diminuer l'agitation lorsque le mélange devient homogène. Eviter la formation de bulles d'air.

## Revendications

1. Utilisation non-thérapeutique d'au moins un sophorolipide, plus particulièrement en cosmétologie, en tant qu'agent stimulateur du métabolisme des fibroblastes du derme de la peau.'

2. Utilisation d'un sophorolipide selon la revendication 1 en tant qu'agent restructurant de la peau.

3. Utilisation d'un sophorolipide selon la revendication 1 en tant qu'agent réparateur de la peau.

4. Utilisation d'un sophorolipide selon la revendication 1 en tant qu'agent raffermissant de la peau.

5. Utilisation d'un sophorolipide selon l'une des revendications 1 à 4 dans laquelle ledit sophorolipide est sous la forme brute.

6. Utilisation d'un sophorolipide selon l'une des revendications 1 à 4 dans laquelle ledit sophorolipide est sous la forme acide.

7. Utilisation d'un sophorolipide selon la revendication 6 dans laquelle ledit sophorolipide a sa fonction acide au moins en partie estérifiée.

8. Utilisation d'un sophorolipide selon l'une des revendications 1 à 5 dans une formulation contenant une concentration de 0,05 ppm à 6 % en poids sur volume, en matière active.

9. Utilisation d'un sophorolipide selon l'une des revendications 1 à 4 et 6 à 7 dans une formulation contenant de 1 ppm à 10 % en poids sur volume, en matière active.

10. Utilisation selon l'une des revendications 1 à 9, sous forme d'émulsions, gels ou sérums.

## Patentansprüche

1. Nicht-therapeutische Verwendung wenigstens eines Sophorolipids, insbesondere bei der Kosmetologie als stimulierendes Reagenz des Metabolismus von Fibroplasten des Coriums der Haut.

2. Verwendung eines Sophorolipids nach Anspruch 1 als restrukturierendes Reagenz der Haut.

3. Verwendung eines Sophorolipids nach Anspruch 1 als reparierendes Reagenz der Haut.

4. Verwendung eines Sophorolipids nach Anspruch 1 als straffendes Reagenz der Haut.

5. Verwendung eines Sophorolipids nach einem der Ansprüche 1 bis 4, bei der das Sophorolipid in Rohform vorliegt.

6. Verwendung eines Sophorolipids nach einem der Ansprüche 1 bis 4, bei der das Sophorolipid in Säureform vorliegt.

7. Verwendung eines Sophorolipids nach Anspruch 6, bei der das Sophorolipid seine saure Funktion wenigstens teilweise esterifiziert hat.

8. Verwendung eines Sophorolipids nach einem der Ansprüche 1 bis 5 in einer Formulierung, welche eine Konzentration von 0,05 ppm bis 6% an Gewicht pro Volumen an aktivem Material enthält.

9. Verwendung eines Sophorolipids nach einem der Ansprüche 1 bis 4 und 6 bis 7 in einer Formulierung, die 1 ppm bis 10 % an Gewicht pro Volumen an aktivem Material enthält.

10. Verwendung nach einem der Ansprüche 1 bis 9 in Form von Emulsionen, Gelen oder Seren.

## Claims

1. Non-therapeutic use of at least one sophorolipid, more particularly in cosmetology as an agent for stimulating the metabolism of fibroblasts of the dermis of the skin.

2. Use of a sophorolipid according to claim 1 as an agent for restructuring the skin.

3. Use of a sophorolipid according to claim 1 as an agent for repairing the skin.

4. Use of a sophorolipid according to claim 1 as an agent for toning up the skin.

5. Use of a sophorolipid according to one of claims 1 to 4, wherein said sophorolipid is in the raw form.

6. Use of a sophorolipid according to one of claims 1 to 4, wherein said sophorolipid is in the acid form.

7. Use of a sophorolipid according to claim 6, wherein said sophorolipid has the acid function which is at least in part esterified.

8. Use of a sophorolipid according to one of claims 1 to 5 in a formulation that contains a concentration of 30 ppm at 6 % by weight to volume, of active ingredient.

9. Use of a sophorolipid according to one of claims 1 to 4 and 6 to 7 in a formulation that contains 0,05 ppm at 10% by weight to volume, of active ingredient.

10. Use according to one of claims 1 to 9, in the form of emulsions, gels, or serums.
